(19) Europäisches Patentamt
European Patent Office
Office européen des brevets

(11) Veröffentlichungsnummer: **0 183 137**
**B1**

# (12) EUROPÄISCHE PATENTSCHRIFT

(45) Veröffentlichungstag der Patentschrift: **11.05.88**

(51) Int. Cl.[4]: **C 07 C 33/12**, C 07 C 29/14, C 07 C 45/45, C 07 C 47/225, A 61 K 7/46, C 11 B 9/00

(21) Anmeldenummer: **85114442.8**

(22) Anmeldetag: **13.11.85**

## (54) 4-(2,2,3-Trimethylcyclopent-3-en-1-yl)-but-3-en-1-ole, Verfahren zur deren Herstellung und Verwendung als Riechstoffe.

(30) Priorität: **16.11.84 DE 3441902**

(43) Veröffentlichungstag der Anmeldung: **04.06.86 Patentblatt 86/23**

(45) Bekanntmachung des Hinweises auf die Patenterteilung: **11.05.88 Patentblatt 88/19**

(84) Benannte Vertragsstaaten: **CH DE FR GB LI NL**

(56) Entgegenhaltungen:
**GB - A - 2 024 208**
**US - A - 4 173 585**

(73) Patentinhaber: **Dragoco Gerberding & Co. GmbH, Dragocostrasse 1, D-3450 Holzminden (DE)**

(72) Erfinder: **Brunke, Ernst-Joachim, Holbeinstrasse 6, D-3450 Holzminden (DE)**
Erfinder: **Kappey, Claus-Hermann, Schratweg 1, D-3450 Holzminden (DE)**

(74) Vertreter: **Patentanwälte Müller-Boré, Deufel, Schön, Hertel, Lewald, Otto, Postfach 26 02 47 Isartorplatz 6, D-8000 München 26 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 183 137 B1

ACTORUM AG

**Beschreibung**

Derivate des Campholenaldehyds (1), einem Umlagerungsprodukt von α-Pinenepoxid, sind als Riechstoffe vom Sandelholztyp bekannt (Übersicht: E.-J. Brunke und E. Klein, in: Fragrance Chemistry, ed. E. Theimer, Academic Press, New York 1982, S. 424 - 26). So sind in der DOS 1 922 391 die durch Aldolkondensation von Campholenaldehyd (1) mit Aceton, Propion- oder Butyraldehyd (unter Katalyse durch Alkalihydroxide oder -alkoholate) erhaltenen α,β-ungesättigten Aldehyde und die daraus durch anschliessende Reduktion erhaltenen α,β-ungesättigten Alkohole 2, 3 und 4 als Riechstoffe mit Sandelholz- und

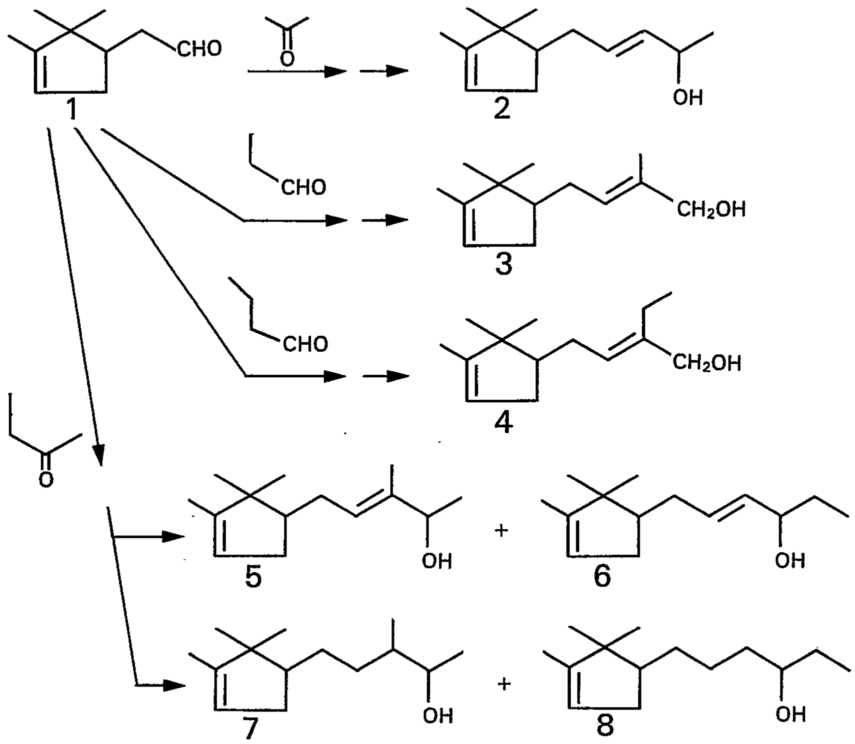
1, 2, 3, 4, 5 + 6, 7 + 8

Mochus-Noten beschrieben worden. Die US-PS 4 052 341 beschreibt die Aldolkondensation von Campholenaldehyd (1) mit Methylethylketon (unter Katalyse durch Alkalihydroxide) zu den α,β-ungesättigten Carbonylverbindungen und deren anschliessende Reduktion zum Gemisch der ungesättigten sekundären Alkohole 5/6 bzw. der gesättigten Alkohole 7/8. In der US-PS 4 173 585 wird die Aldolkondensation von Campholenaldehyd mit verschiedenen Ketonen unter Verwendung von Zinkacetat-dihydrat beschrieben. Hierbei entstehen im Gegensatz zu den Aldolkondensationen unter Katalyse mit Alkalihydroxiden oder -alkoholaten nicht die einheitlichen α,β-ungesättigten Ketone, sondern vielmehr Gemische von α,β- und β,γ-ungesättigten Ketonen, die als (Z)- und (E)-Isomere vorliegen (9-12). Diese Gemische sollen Geruchsnoten vom Jonon-Typ

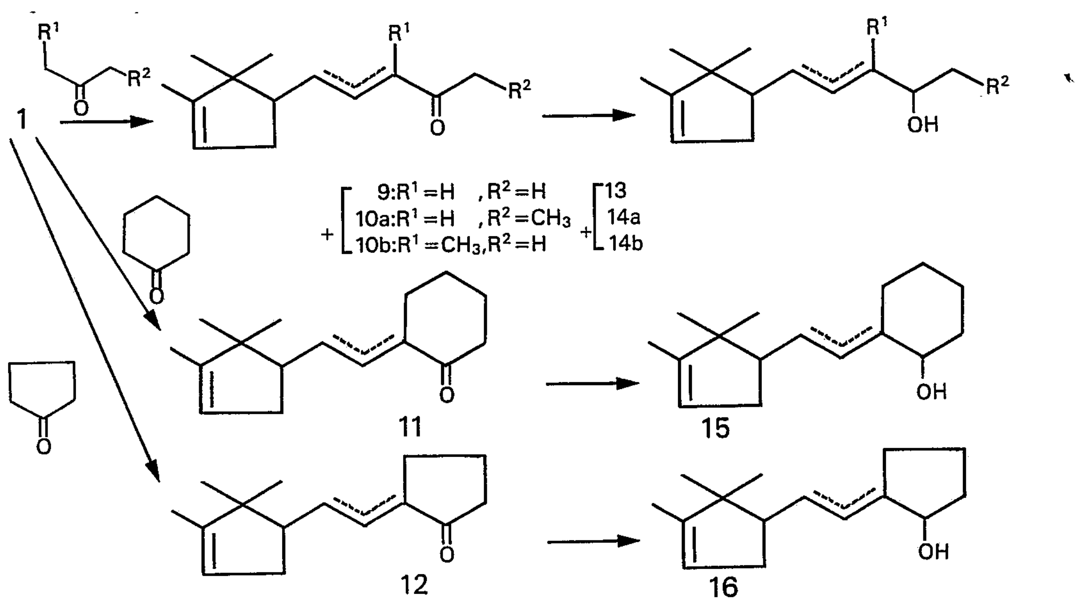

1, 11, 15, 12, 16

besitzen (süss, holzig, grün, Melonen-artig, Aprikose-artig, blumig, Veilchen-artig, Ambra-artig). Die aus den Gemischen der α,β- und β,γ-ungesättigten Ketone durch Reduktion erhaltenen Alkoholgemische 14-16 sollen Sandelholz-Cedernholz-, Harz- und Blumen-Noten besitzen. Die einzelnen Isomeren wurden nicht isoliert. Über die Geruchseigenschaften der einzelnen Isomeren ist nichts bekannt.

Wir haben nun überraschenderweise gefunden, dass die bisher nicht bekannten β,γ-ungesättigten Alkohole der allgemeinen Formel A charakteristische holzig-animalische und weich-fruchtige Geruchseigenschaften besitzen, die sich wesentlich von der typischen Sandelholz- bzw. Moschusnote der α,β-ungesättigten Alkohole 3 oder 4 unterscheiden.

Die Geruchseigenschaften der Verbindungen der allgemeinen Formel A ermögliche vorteilhaft ihre Verwendung als Riechstoffe, insbesonders in Kombination mit den jeweils α,β-ungesättigten Alkoholen gleicher Substitution.

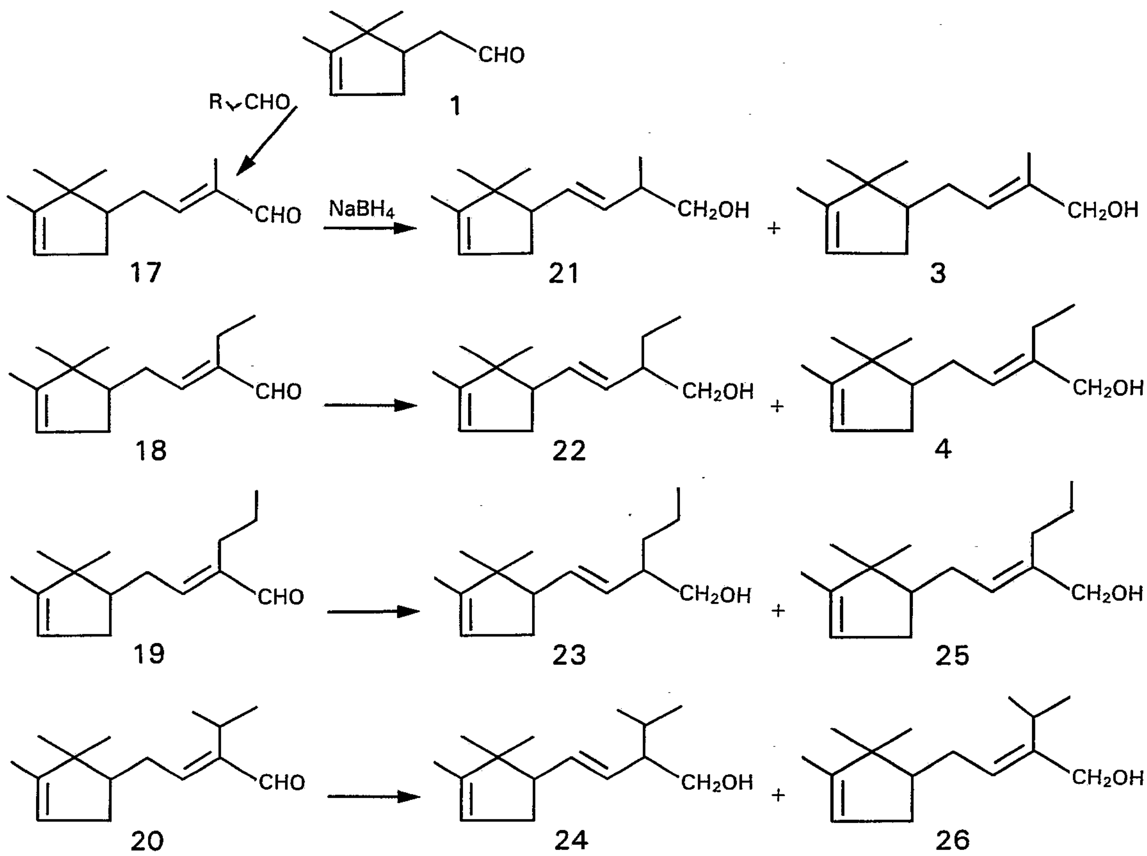

1
17 21 3
18 22 4
19 23 25
20 24 26

Für die Herstellung der β,γ-ungesättigten Alkohole der allgemeinen Formel A wurde ebenfalls von Campholenaldehyd (1) ausgegangen, der in bekannter Weise mit kurzkettigen aliphatischen Aldehyden kondensiert wurde, wobei die α,β-ungesättigten Aldehyde 17 - 20 entstanden. Vorschriften für diese Aldolkondensationen sind in der DOS 1 922 391 und in dem US-Pat. 4 052 341 gegeben worden. Die Reduktion der α,β-ungesättigten Aldehyde 17 und 18 zu den entsprechenden α,β-ungesättigten Alkoholen 3 und 4 kann nach der DOS 1 922 391 mit komplexen Metallhydriden oder in Gegenwart von Aluminiumalkoholaten nach der Methode von Meerwein-Pondorf erfolgen. Wir haben nun überraschenderweise festgestellt, dass bei der Verwendung komplexer Hydride, vorzugsweise Natriumborhydrid, in Gegenwart eines basischen Mediums, vorzugsweise Natronlauge, eine partielle Dekonjugierung der Doppelbindung von der α,β- in die β,γ-Position erfolgt, wobei das sich einstellende Gleichgewicht durch die Temperatur und die Reaktionszeit, aber auch durch die Reduktion der Carbonylgruppe zur Alkoholgruppe beeinflusst wird (Beispiel 1, Tabelle 1). Aus den Produktgemischen können die Verbindungen der Formel A durch an und für sich bekannte Trennverfahren isoliert werden, vorzugsweise durch Destillation.

Die für die neuen Verbindungen der allgemeinen Formel A angegebene Struktur kann durch spektroskopische Methoden bewiesen werden. Im $^1H$-NMR-Spektrum (Abb. 1) des isolierten Alkohols 22 erscheint neben dem Signal für das olefinische Proton des Cyclopenten-Systems ein Multiplett bei $\delta = 4.9 - 5.7$ ppm (2 Protonen), das die β,γ-Doppelbindung in der Seitenkette kennzeichnet. Diese Lage der Doppelbindung von 22 wird ebenfalls durch eine starke IR-Bande bei 968 $cm^{-1}$ (disubstituierte Doppelbindung) aufgezeigt. Die massenspektrometrische Fragmentierung von 22 steht ebenfalls in Übereinstimmung mit der angegebenen Struktur; die für (E-22) angegebenen Fragmente (Abb. 2) treten in sehr ähnlicher Form auch bei dem Z-konfigurierten 22 auf (Abb. 3). Der auffälligste massenspektroskopische Unterschied zu den entsprechenden α,β-ungesättigten Alkoholen besteht für die β,γ-ungesättigten Alkohole in dem hierfür günstigen α,β-Bindungsbruch, der zu einem stärkeren Fragment-Ion mit m/z = 135 führt (Massenspektrum von 4: Abb. 4). Bei

den Allylalkoholen wie 4, das aus 18 durch Reduktion mit Lithiumaluminiumhydrid (in Diethylether) dargestellt wurde, ist das durch Allylspaltung entstehende Fragment mit m/z = 109 (Cyclopentensystem) wesentlich stärker ausgeprägt als bei den entsprechenden β,γ-ungesättigten Alkoholen (hier: 22). Ausserdem tritt bei den β,γ-ungesättigten Verbindungen ein typisches Fragment-Ion mit m/z = $M^+$-31 auf (s. Abb. 2), das bei den entsprechenden α,β-ungesättigten Alkoholen eine deutlich geringere Intensität aufweist (Abb. 4). Ein weiterer Konstitutionsbeweis ist durch die selektive Hydrierung gegeben, die zu den analogen Verbindungen mit gesättigter Seitenkette führte; diese Verbindungen sind in der DE-PS 2 827 957 beschrieben worden. Die genannten Kriterien beweisen die Struktur der neuen Verbindungen der Formel A.

Bei Verwendung von Lithiumaluminiumhydrid/Ether (0 °C) zur Reduktion der ungesättigten Aldehyde 17-20 erfolgt unter üblichen Reaktionsbedingungen keine Dekonjugation.

*Beispiel 1*

*Reduktion von 2-Ethyl-4-(2,2,3-trimethylcyclopent-3-en-1-yl)-but-2-en-al*

Zu einer gerühren Lösung von 257,5 g (1,25 Mol) 2-Ethyl-4-(2,2,3-trimethylcyclopent-3-en-1-yl)-but-2-en-al (18, dargestellt analog US-PS 4 052 341) in 250 g Methanol wurde bei 60 °C innerhalb 1 Stunde eine Lösung von 15 g (0,395 mol) Natriumborhydrid und 0,15 g Natriumhydroxid in 50 g Wasser getropft.

Die Reaktionslösung wurde weitere 30 min bei 60 °C belassen. Dann wurde das Methanol abdestilliert. Der verbleibende Rückstand wurde in 200 g Petrolether (Sdp. 50 - 70 °C) aufgenommen und mit Natriumchloridlösung neutralgewaschen. Der Petrolether wurde destillativ entfernt, und das zurückbleibende Rohprodukt fraktioniert destilliert, wobei ein Gemisch von 2-Ethyl-4-(2,2,3-trimethylcyclopent-3-en-1-yl)-but-3-en-1-ol (22, E + Z-Isomer), 2-Ethyl-4-(2,2,3-trimethylcyclopent-3-en-1-yl)-butan-1-ol und 2-Ethyl-4-(2,2,3-trimethylcyclopent-3-en-1-yl)-but-2-en-1-ol (4, E + Z-Isomer) anfiel: Die Ausbeute betrug 203 g (78%); Sdp. 110-124 °C/2 Pa; Gaschromatogramm Abb. 5; Massenspektrum für E-22 Abb. 2, für Z-22 Abb. 3 und E-4 Abb.4.

Ein kleiner Teil des vorstehenden destillierten Alkoholgemisches wurde an einer 1 m-Drehbandkolonne erneut fraktioniert destilliert, wobei eine analytische Probe von reinem 2-Ethyl-4-(2,2,3-trimethylcyclopent-3-en-1-yl)-but-3-en-1-ol (22, E + Z-Isomer) anfiel: Sdp. 111-113 °C/2 Pa; $^1$H-NMR-Spektrum Abb. 1.

Die Reaktion wurde analog zu der hier gegebenen Vorschrift bei anderen Temperaturen und mit anderen Reaktionszeiten ausgeführt. Die Produktgemische wurden gaschromatographisch untersucht (30 m DB WAX-30N; Temperaturprogramm: 100 bis 240 °C, 4 °C/min). Die Ergebnisse sind in der Tabelle 1 zusammengefasst.

Tabelle 1: *Zusammensetzung der Reduktionssprodukte von 18*

| Bedingungen | dekonjugierte Alkohole %Z | %E | gesättigt. Alkohol (%) | konjugierte Alkohole %Z | %E | Verhältnis E/Z | Verhältnis konj./dekonj. |
|---|---|---|---|---|---|---|---|
| 15 °C/5 min | 1,95 | 2,13 | 2,17 | 6,10 | 87,64 | 14,37 | 22,98 |
| 30 °C/5 min | 1,92 | 2,14 | 2,55 | 5,99 | 87,39 | 14,59 | 23,00 |
| 40 °C/5 min | 2,30 | 2,44 | 3,04 | 6,16 | 86,06 | 13,97 | 19,46 |
| 60 °C/5 min | 3,71 | 4,05 | 3,23 | 6,48 | 82,51 | 12,73 | 11,45 |
| 30 °C/1 h | 4,03 | 4,44 | 3,57 | 6,96 | 81,01 | 11,64 | 10,39 |
| 60 °C/1 h | 7,95 | 9,12 | 2,68 | 7,98 | 72,27 | 9,06 | 4,70 |
| 10 °C/45 min *invers* | 2,66 | 2,95 | 2,66 | 6,98 | 84,75 | 12,15 | 16,33 |

*Beispiel 2*

*Reduktion von 2-Methyl-4-(2,2,3-trimethyl-cyclopent-3-en-1-yl)-but-2-en-al*

Zu einer gerührten Lösung von 153,6 g (0,8 mol) 2-Methyl-4-(2,2,3-trimethylcyclopent-3-en-1-yl)-but-2-en-al (17, dargestellt analog US-PS 4 052 341) in 160 g Methanol wurde bei 60 °C innerhalb 1 h eine Lösung von 10 g (0,236 mol) Natriumborhydrid und 0,1 g Natriumhydroxid in 33 g Wasser getropft. Die Reaktionslösung wurde weitere 30 min bei 60 °C belassen. Dann wurde das Methanol abdestilliert.

Der verbleibende Rückstand wurde in 150 g Petrolether (Sdp. 50-70 °C) aufgenommen und mit Natriumchloridlösung neutralgewaschen. Der Petrolether wurde destillativ entfernt, und das verbleibende Rohprodukt fraktioniert destilliert, wobei ein Gemisch von 2-Methyl-4-(2,2,3-trimethylcyclopent-3-en-1-yl)-but-3-en-1-ol (21, E + Z-Isomer), 2-Methyl-4-(2,2,3-trimethylcyclopent-3-en-1-yl)-butan-1-ol und 2-Methyl-4-(2,2,3-trimethylcyclopent-3-en-1-yl)-but-2-en-1-ol (3, E + Z-Isomer) anfiel: Die Ausbeute betrug 115 g (74%); Sdp. 96-110 °C/2 Pa; Gaschromatogramm Abb. 6.

Die Reaktion wurde analog zu der hier gegebenen Vorschrift bei anderen Temperaturen und mit anderen Reaktionszeiten ausgeführt. Die Produktgemische wurden gaschromatographisch untersucht (30 m DB WAX-30N; Temperaturprogramm 100 - 240 °C, 4 °C/min). Die Ergebnisse sind in der Tabelle 2 zusammengefasst.

Tabelle 2: *Zusammensetzung der Reduktionssprodukte von 17*

| Bedingungen | dekonjugierte Alkohole E+Z (%) | gesättigt. Alkohol (%) | konjugierte Alkohole %Z | %E | Verhältnis E/Z | Verhältnis konj./dekonj. |
|---|---|---|---|---|---|---|
| 30°C/5 min | 2,07 | 3,29 | 3,97 | 90,78 | 22,89 | 45,82 |
| 60°C/5 min | 4,79 | 3,91 | 4,41 | 86,90 | 19,70 | 19,04 |
| 30°C/1 h | 2,14 | 3,16 | 4,10 | 90,60 | 22,09 | 44,34 |
| 60°C/1 h | 6,42 | 3,55 | 4,36 | 85,67 | 19,66 | 14,02 |

*Beispiel 3*

*Massenspektrometrische Daten*

Z-21

| m/z | (%) | m/z | (%) | m/z | (%) |
|---|---|---|---|---|---|
| 39 | 23 | 79 | 37 | 108 | 64 |
| 41 | 57 | 83 | 31 | 109 | 25 |
| 43 | 31 | 91 | 51 | 119 | 36 |
| 55 | 63 | 93 | 100 | 121 | 85 |
| 67 | 30 | 95 | 22 | 124 | 48 |
| 69 | 37 | 105 | 38 | 135 | 33 |
| 77 | 36 | 107 | 74 | $M^+$: 194 | 6 |

E-21

| m/z | (%) | m/z | (%) | m/z | (%) |
|---|---|---|---|---|---|
| 41 | 43 | 79 | 32 | 108 | 57 |
| 43 | 30 | 83 | 20 | 109 | 22 |
| 55 | 56 | 91 | 44 | 119 | 38 |
| 67 | 30 | 93 | 100 | 121 | 45 |
| 69 | 34 | 105 | 28 | 124 | 36 |
| 77 | 28 | 107 | 83 | 135 | 26 |
| | | | | $M^+$: 194 | 1 |

Z-3

| m/z | (%) | m/z | (%) | m/z | (%) |
|---|---|---|---|---|---|
| 39 | 21 | 79 | 40 | 107 | 54 |
| 41 | 51 | 81 | 27 | 108 | 100 |
| 43 | 53 | 84 | 41 | 109 | 62 |
| 53 | 20 | 91 | 40 | 119 | 29 |
| 55 | 36 | 93 | 87 | 121 | 54 |
| 67 | 72 | 95 | 47 | 122 | 25 |
| 77 | 27 | 105 | 32 | 161 | 28 |
| | | | | $M^+$: 194 | 6 |

E-3

| m/z | (%) | m/z | (%) | m/z | (%) |
|---|---|---|---|---|---|
| 39 | 24 | 79 | 64 | 108 | 100 |
| 41 | 42 | 81 | 37 | 109 | 95 |
| 43 | 45 | 91 | 62 | 119 | 27 |
| 53 | 26 | 93 | 86 | 121 | 96 |
| 55 | 31 | 94 | 23 | 122 | 49 |
| 65 | 21 | 95 | 91 | 161 | 45 |
| 67 | 84 | 105 | 33 | 179 | 26 |
| 77 | 49 | 107 | 90 | $M^+$: 194 | 12 |

Z-22

| m/z | (%) | m/z | (%) | m/z | (%) |
|---|---|---|---|---|---|
| 30 | 31 | 77 | 39 | 108 | 59 |
| 39 | 17 | 79 | 46 | 109 | 46 |
| 41 | 54 | 81 | 21 | 119 | 31 |
| 43 | 22 | 83 | 41 | 121 | 100 |
| 53 | 19 | 91 | 59 | 122 | 21 |
| 55 | 45 | 93 | 82 | 133 | 17 |
| 57 | 21 | 95 | 32 | 135 | 44 |
| 65 | 16 | 105 | 45 | 138 | 36 |
| 67 | 30 | 107 | 87 | 177 | 20 |
| | | | | $M^2$: 208 | 4 |

E-22

| m/z | (%) | m/z | (%) | m/z | (%) |
|---|---|---|---|---|---|
| 29 | 15 | 69 | 45 | 108 | 71 |
| 31 | 29 | 77 | 40 | 109 | 42 |
| 39 | 19 | 79 | 41 | 119 | 39 |
| 41 | 51 | 81 | 21 | 121 | 100 |
| 43 | 20 | 83 | 36 | 122 | 22 |
| 53 | 18 | 91 | 61 | 133 | 18 |
| 55 | 44 | 93 | 89 | 135 | 44 |
| 57 | 22 | 95 | 34 | 138 | 39 |
| 65 | 16 | 105 | 46 | 177 | 23 |
| 67 | 30 | 107 | 88 | $M^+$: 208 | 5 |

Z-4

| m/z | (%) | m/z | (%) | m/z | (%) |
|---|---|---|---|---|---|
| 41 | 39 | 79 | 31 | 107 | 47 |
| 43 | 27 | 81 | 23 | 108 | 100 |
| 55 | 29 | 91 | 26 | 109 | 56 |
| 57 | 38 | 93 | 63 | 119 | 21 |
| 67 | 45 | 95 | 33 | 121 | 46 |
| 77 | 26 | 98 | 26 | 122 | 21 |
| | | | | $M^+$: 208 | 5 |

E-4

| m/z | (%) | m/z | (%) | m/z | (%) |
|---|---|---|---|---|---|
| 27 | 14 | 77 | 36 | 108 | 98 |
| 29 | 28 | 79 | 52 | 109 | 97 |
| 30 | 11 | 81 | 42 | 119 | 29 |
| 39 | 23 | 82 | 15 | 121 | 100 |
| 41 | 56 | 83 | 13 | 122 | 43 |
| 43 | 49 | 91 | 45 | 123 | 11 |
| 53 | 24 | 93 | 79 | 135 | 18 |

E-4

| m/z | (%) | m/z | (%) | m/z | (%) |
|---|---|---|---|---|---|
| 55 | 45 | 94 | 16 | 161 | 13 |
| 57 | 81 | 95 | 77 | 175 | 27 |
| 65 | 15 | 98 | 15 | 177 | 11 |
| 67 | 82 | 105 | 25 | 193 | 15 |
| 69 | 18 | 107 | 80 | $M^+$: 208 | 10 |

Z-23

| m/z | (%) | m/z | (%) | m/z | (%) |
|---|---|---|---|---|---|
| 31 | 28 | 83 | 41 | 109 | 47 |
| 40 | 65 | 91 | 53 | 110 | 31 |
| 43 | 29 | 93 | 71 | 119 | 41 |
| 55 | 67 | 95 | 35 | 121 | 100 |
| 67 | 35 | 105 | 35 | 122 | 28 |
| 77 | 31 | 107 | 82 | 135 | 47 |
| 79 | 39 | 108 | 73 | 152 | 33 |
| 81 | 26 | | | $M^+$: 222 | 8 |

E-23

| m/z | (%) | m/z | (%) | m/z | (%) |
|---|---|---|---|---|---|
| 31 | 25 | 83 | 44 | 109 | 60 |
| 40 | 52 | 91 | 42 | 110 | 27 |
| 43 | 29 | 93 | 96 | 119 | 42 |
| 55 | 79 | 95 | 29 | 121 | 100 |
| 67 | 33 | 105 | 42 | 135 | 54 |
| 69 | 25 | 107 | 75 | 152 | 29 |
| 77 | 29 | 108 | 77 | 191 | 27 |
| 79 | 48 | | | $M^+$: 222 | 10 |

Z-25

| m/z | (%) | m/z | (%) | m/z | (%) |
|---|---|---|---|---|---|
| 29 | 26 | 71 | 42 | 95 | 74 |
| 39 | 26 | 77 | 38 | 105 | 26 |
| 41 | 74 | 79 | 68 | 107 | 76 |
| 43 | 49 | 81 | 35 | 108 | 88 |
| 53 | 31 | 91 | 51 | 109 | 100 |
| 55 | 45 | 93 | 76 | 121 | 98 |
| 67 | 78 | 94 | 26 | 122 | 49 |
| | | | | $M^+$: 222 | 10 |

E-25

| m/z | (%) | m/z | (%) | m/z | (%) |
|---|---|---|---|---|---|
| 39 | 22 | 77 | 27 | 107 | 35 |
| 40 | 51 | 79 | 49 | 108 | 38 |
| 43 | 54 | 81 | 30 | 109 | 100 |
| 55 | 41 | 91 | 27 | 119 | 24 |
| 57 | 22 | 93 | 65 | 121 | 43 |
| 67 | 49 | 94 | 22 | 122 | 22 |
| 69 | 22 | 95 | 76 | 189 | 22 |
| 71 | 60 | | | $M^+$: 222 | 8 |

Z-24

| m/z | (%) | m/z | (%) | m/z | (%) |
|---|---|---|---|---|---|
| 31 | 28 | 81 | 30 | 109 | 92 |
| 41 | 72 | 83 | 57 | 112 | 34 |

Z-24

| m/z | (%) | m/z | (%) | m/z | (%) |
|---|---|---|---|---|---|
| 43 | 55 | 91 | 70 | 119 | 49 |
| 55 | 98 | 93 | 89 | 121 | 100 |
| 67 | 40 | 95 | 51 | 122 | 30 |
| 69 | 47 | 105 | 57 | 133 | 30 |
| 77 | 43 | 107 | 77 | 135 | 62 |
| 79 | 45 | 108 | 87 | $M^+$: 222 | 11 |

E-24

| m/z | (%) | m/z | (%) | m/z | (%) |
|---|---|---|---|---|---|
| 31 | 28 | 77 | 44 | 107 | 74 |
| 39 | 26 | 79 | 50 | 108 | 80 |
| 41 | 76 | 81 | 28 | 109 | 98 |
| 43 | 46 | 83 | 52 | 112 | 32 |
| 53 | 26 | 91 | 57 | 119 | 50 |
| 55 | 100 | 93 | 85 | 121 | 98 |
| 67 | 43 | 95 | 48 | 133 | 26 |
| 69 | 44 | 105 | 57 | 135 | 63 |
| | | | | $M^+$: 222 | 7 |

Z-26

| m/z | (%) | m/z | (%) | m/z | (%) |
|---|---|---|---|---|---|
| 41 | 37 | 81 | 19 | 108 | 100 |
| 43 | 37 | 91 | 22 | 109 | 52 |
| 55 | 33 | 93 | 61 | 112 | 16 |
| 67 | 36 | 95 | 31 | 119 | 18 |
| 69 | 19 | 105 | 18 | 121 | 42 |
| 77 | 24 | 107 | 39 | 122 | 17 |
| 79 | 30 | | | $M^+$: 222 | 4 |

E-26

| m/z | (%) | m/z | (%) | m/z | (%) |
|---|---|---|---|---|---|
| 39 | 28 | 71 | 26 | 95 | 68 |
| 41 | 68 | 77 | 40 | 107 | 75 |
| 43 | 60 | 79 | 75 | 108 | 100 |
| 53 | 28 | 81 | 39 | 109 | 95 |
| 55 | 49 | 91 | 52 | 121 | 93 |
| 67 | 65 | 93 | 83 | 122 | 40 |
| 69 | 26 | 94 | 28 | $M^+$: 222 | 6 |

Der Campholenaldehyd (1) kann aus (+)- oder (−)-α-Pinen in optisch aktiver Form hergestellt werden. Somit kann die C-1-Seitenkette der Verbindungen A α- oder β-konfiguriert sein. Die dekonjugierten Alkohole der Formel A weisen in α-Position (Verzweigung der Seitenkette) ein zusätzliche Chiralitätszentrum auf. Somit liegen die Alkohole A als Diastereomere vor; die angegebene Z/E-Isomerie kann zusätzlich vorliegen. Die hier als (Z)- bzw. (E)-Isomere zugeordneten β,γ-ungesättigten Alkohole können daher auch bei gleicher Geometrie als Diastereomere vorliegen.

*Beispiel 4*

*Parfüm-Base süss-krautigen Typs*

| | |
|---|---|
| 2,2-Dimethyl-3-phenyl-propanol (Muguetalkohol, DE-PS 3 139 358) | 50 |
| Lavendelöl, französisch | 15 |
| Ysopöl, spanisch | 10 |

| | |
|---|---|
| Eichenmoos-Extrakt, 50%ig in Dipropylenglykol | 10 |
| Dihydromyrcenalkohol | 5 |
| Rosmarinöl, spanisch | 5 |
| Produkt aus Beispiel 2 (21 + 3) | 5 |
| | 100 |

Diese Base, die einen relativ hohen Anteil an dem Produkt aus Beispiel 2 enthält, besitzt einen ansprechenden frischen und süss-krautigen Akkord mit einer deutlichen, aber weichen Holznote.

*Beispiel 5*

*Parfüm-Base holzig-würzigen Typs*

| | |
|---|---|
| Mahagonat | 150 |
| Vetiverylacetat | 100 |
| Muscogene | 100 |
| Caryophyllenol | 100 |
| Patchouli-Öl | 50 |
| 2,2-Dimethyl-5-phenylpropanol (Muguetalkohol, DE-PS 3 139 358) | 50 |
| Moschus Keton | 30 |
| Eichenmoos-Extrakt, 50%ig | 20 |
| Labdanum-Resinoid, 50%ig | 20 |
| Myrrhe-Resinoid | 10 |
| Pfefferöl | 5 |
| Muskatnussöl | 5 |
| Cistusöl | 5 |
| Eugenol | 5 |
| | 650 |

Diese Parfümbase besitzt einen holzig-würzigen Charakter mit Moschus-Aspekten. Bei Zugabe von 100 Teilen der Verbindung 4 (DE-OS 1 022 391) ist eine Abrundung unter gleichzeitiger Betonung süsslich-holziger Aspekte festzustellen. Gibt man 100 Teile der Verbindung 22 hinzu, so wird der Geruchseindruck harmonischer unter Betonung der holzig-animalischen Aspekte. Eine Einarbeitung von 100 Teilen des Produkts aus Beispiel 1 (22 + 4) ergibt eine ausgewogene Komposition mit Sandelholzaspekt.

*Beispiel 6*

*Parfumöl blumigen Typs*

| | *a* | *b* | *c* |
|---|---|---|---|
| Citronellol | 20 | 20 | 20 |
| Phenylethylalkohol | 170 | 170 | 170 |
| Indol, 50%ig in Dipropylenglykol | 5 | 5 | 5 |
| Isoeugenol | 5 | 5 | 5 |
| Benzylacetat | 30 | 30 | 30 |
| Linalool | 40 | 40 | 40 |
| Terpineol | 240 | 240 | 240 |
| 2,2-Dimethyl-3-phenyl-propanol-1 (Muguetalkohol, DE-PS 3 139 358) | 200 | 200 | 200 |
| Zimtalkohol | 40 | 40 | 40 |
| Citronellylacetat | 20 | 20 | 20 |
| Frambinon | 15 | 15 | 15 |
| 1,4-dimethyl-8-hydroximino-bicyclo[3.2.1]octan (DE-PS 3 129 934) | 60 | 60 | 60 |
| Hydroxycitronellal | 25 | 25 | 25 |
| Verbindung 4 (DE-OS 1 922 391) | 20 | — | — |
| Verbindung 22 | — | 20 | — |
| Produkt aus Beispiel 1 (22+4) | — | — | 20 |
| | 890 | 890 | 890 |

Die Mischung a besitzt einen ausgewogenen blumigen Duft vom Typ Maiglöckchen/weisse Lilie. Bei der Mischung b, die anstelle des Allylalkohols 4 die neue Verbindung 22 enthält ist eine Betonung der frischen Note festzustellen. Die Mischung c wiederum besitzt einen harmonischen Blumenduft mit einer leichten Betonung holziger Aspekte.

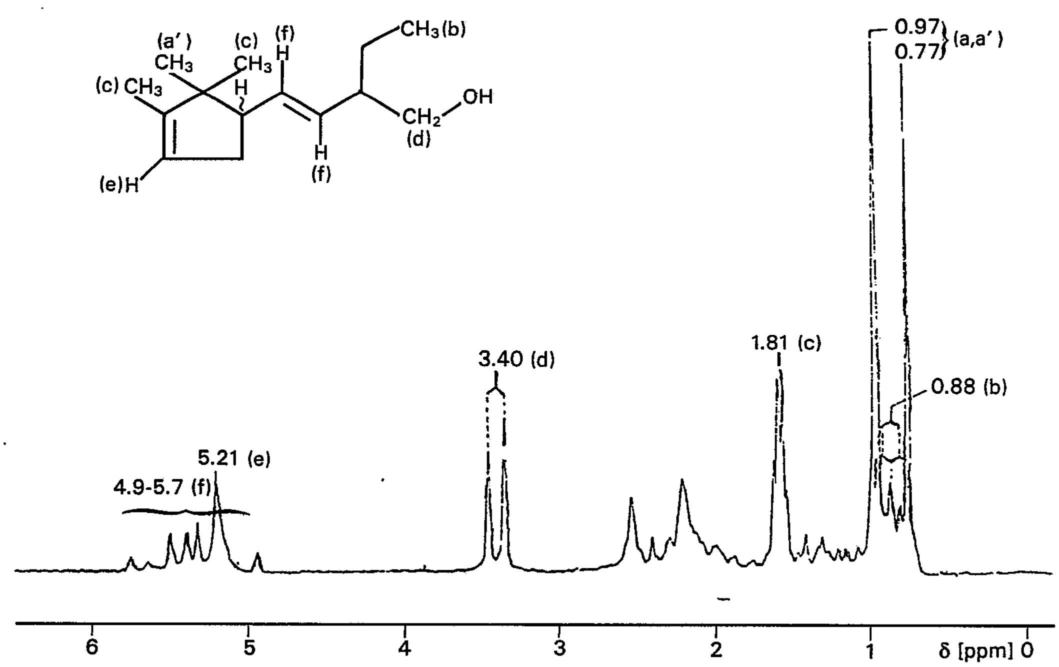


Abb. 1: $^1$H-NMR-Spektrum (60 MHz, $CCl_4$) von 22

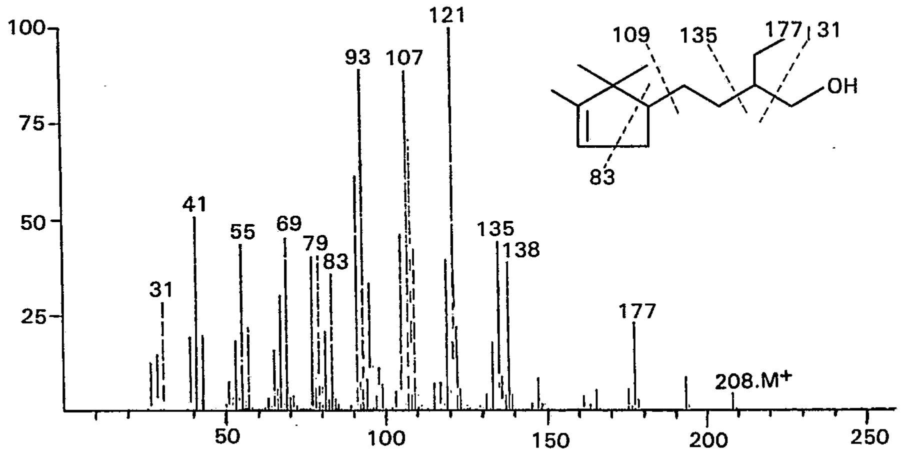


Abb. 2: Massenspektrum von 22 (E)

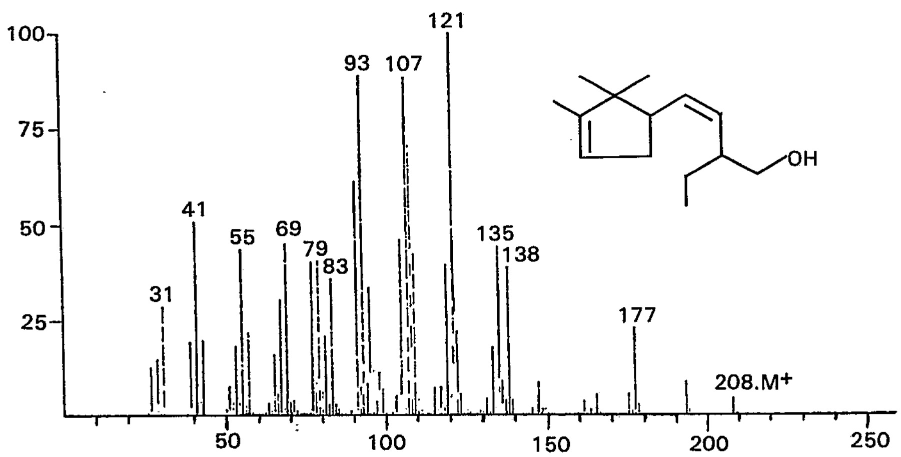


Abb. 3: Massenspektrum von 22 (Z)

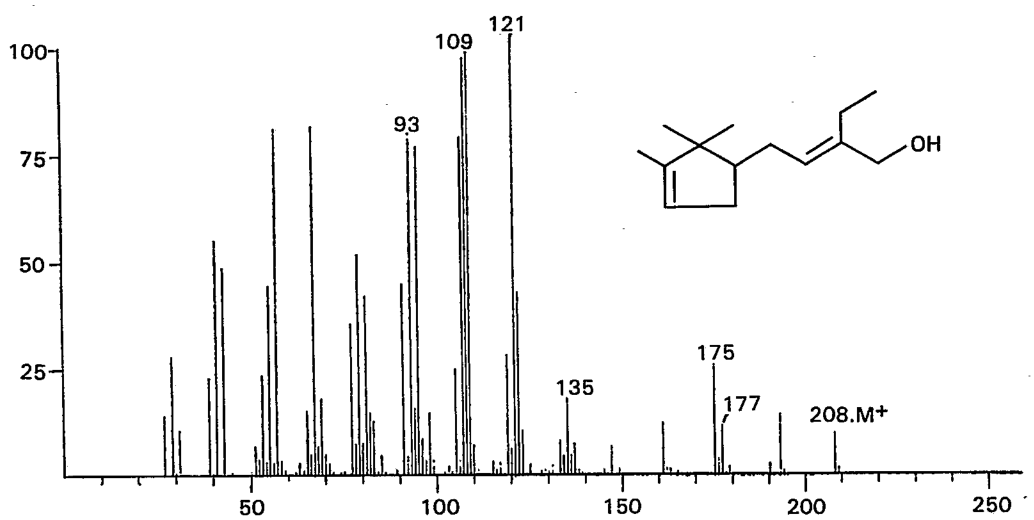


Abb. 4: Massenspektrum von 4 (E)

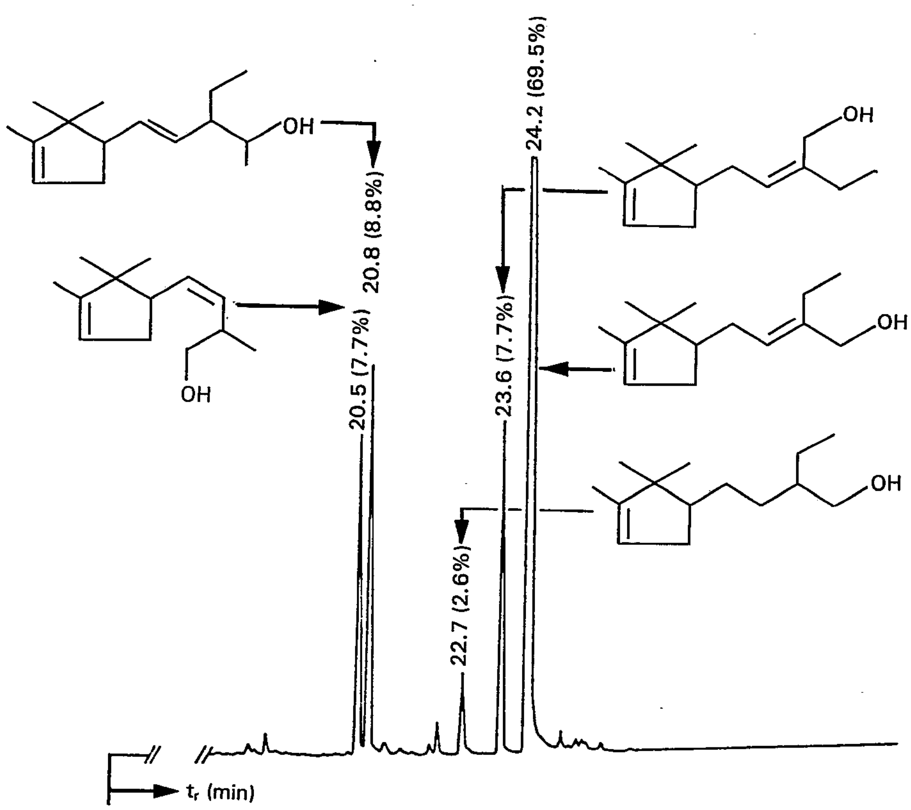


Abb. 5: Gaschromatogramm (30 m DB WAX-30N; Temperaturprogramm 100-240°C, 4°C/min) des Reduktionsgemisches nach Beispiel 1

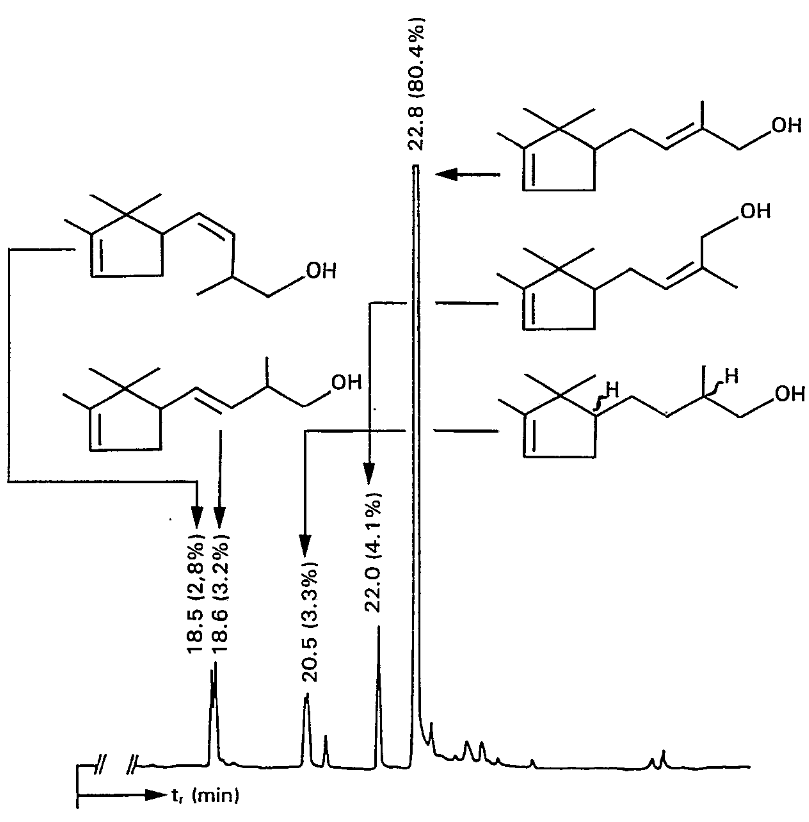


Abb. 6: Gaschromatogramm (30 m DB WAX-30N; Temperaturprogramm 100-240°C, 4°C/min) des Reduktionsgemisches nach Beispiel 2

**Patentansprüche**

1. 4-(2,2,3-Trimethylcyclopent-3-en-1-yl)-but-3-en-1-ole, der allgemeinen Formel A

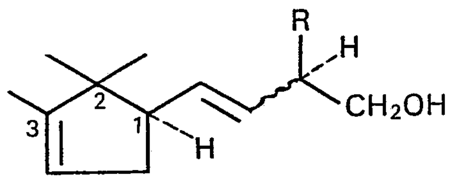

A

R = Me, Et, Pr, i-Pr, Bu

wobei die geschlängelte Linie geometrische Isomere und die gestrichelten Linien stereoisomere Formen kennzeichnen.

2. Verfahren zur Herstellung der Verbindungen der allgemeinen Formel A nach Anspruch 1, dadurch gekennzeichnet, dass man a) Campholenaldehyd und aliphatische Aldehyde in bekannter Weise kondensiert, b) die erhaltenen α,β-ungesättigten Aldehyde unter Verwendung von Natriumhydrid in alkalischem Milieu bei Temperaturen von 10 bis 80°C, vorzugsweise 30 - 60°C, mit erhöhter Reaktionsdauer oder inverser Reaktionsführung reduziert und c) gegebenenfalls aus dem Produktgemisch die Verbindung A durch Destillation abtrennt.

3. Verwendung der Verbindungen der allgemeinen Formel A nach Anspruch 1 als Riechstoffe, bzw. Bestandteile von Parfümölen für kosmetische oder technische Konsumgüter.

4. Verwendung von mindestens 1% an Verbindungen der allgemeinen Formel A enthaltenden Reduktionsgemischen nach Anspruch 2, Stufe b, als Riechstoffe bzw. Bestandteile von Perfümölen für kosmetische oder technische Konsumgüter.

**Claims**

1. 4-(2,2,3-trimethylcyclopent-3-en-1-yl)-but-3-en-1-ols, according to the general formula A

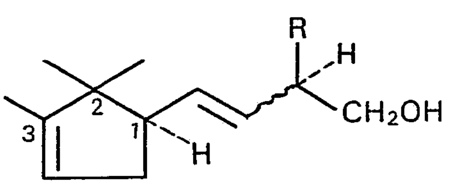

A

R = Me, Et, Pr, i-Pr, Bu

wherein the wavy line characterizes geometrical isomers and the broken lines characterize stereoisomeric forms.

2. A process of producing the compounds of the general formula A according to claim 1, characterized by a) condensing in per se known manner campholene aldehyde and aliphatic aldehydes, b) reducing the resultant alpha,beta-unsaturated aldehydes using sodium hydride in alkaline environment at temperatures of from 10-80°C, preferably from 30 - 60°C, with increased reaction duration or inverse reaction conduction and c) optionally separating the compound A from the product mixture by distillation.

3. The use of the compounds of the general formula A according to claim 1 as aroma substances or, resp., component parts of perfume oils for cosmetic or technical consumers' goods.

4. The use of reduction mixture according to claim 2, step b containing at least 1% of compounds of the general formula A as aroma substances or, resp., component parts of perfume oils for cosmetic or technical consumers' goods.

**Revendications**

1. 4-(2,2,3-triméthylcyclopentène-3 yl-1)-butène-3 ols-1 de formule générale A

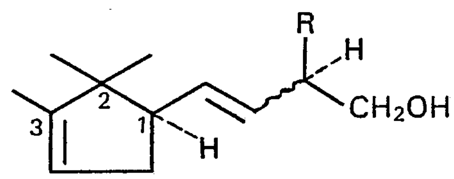

A

R = Me, Et, Pr, i-Pr, Bu

dans laquelle la ligne ondulée représente des isomères géométriques et les lignes en tirets représentent des formes stéreoisomères.

2. Procédé pour la préparation de composés de formule générale A selon la revendication 1, caractérisé en ce que a) on condense d'une manière connue le campholènaldéhyde et des aldéhydes aliphatiques, b) on réduit les aldéhydes α,β-insaturés obtenus, en utilisant de l'hydrure de sodium en milieu alcalin à des températures de 10-80°C, de préférence de 30-60°C, en augmentant la durée de la réaction ou en conduisant la réaction d'une manière inverse, et c) éventuellement, on sépare le composé A du mélange de produits, par distillation.

3. Utilisation des composés de formule générale A selon la revendication 1 en tant que matières parfumantes ou constituants d'huiles de parfum pour produits de consommation cosmétiques ou techniques.

4. Utilisation de mélanges de produits de réduction selon la revendication 2, étape b, contenant au moins 1% de composés de formule générale A, en tant que matières parfumantes ou constituants d'huiles de parfum pour produits de consommation cosmétiques ou techniques.